# FASCICULE DE BREVET EUROPEEN

(11) **EP 2 358 413 B1**
(45) Date de publication et mention de la délivrance du brevet: **22.03.2017**
(21) Numéro de dépôt: 09784079.7
(22) Date de dépôt: 11.12.2009
(51) Int. Cl.: A61M 1/10, A61M 1/36

(54) **DISPOSITIF MEDICAL PULSATILE DESTINE A ETRE UTILISE AVEC LA CHIRURGIE EXTRA-CORPORELLE**
PULSIERENDE MEDIZINISCHE VORRICHTUNG FÜR DEN EINSATZ BEI DER AUSSERKÖRPERLICHEN CHIRURGIE
PULSATILE MEDICAL DEVICE DESIGNED TO BE USED IN EXTRACORPOREAL SURGERY

(30) Priorité: 12.12.2008 FR 0858546
(43) Date de publication de la demande: 24.08.2011
(73) Titulaire: Nour, Sayed, 92370 Chaville (FR); Chastanier, Pierre, 75008 Paris (FR)
(72) Inventeur: Nour, Sayed, 92370 Chaville (FR)
(74) Mandataire: Thomas, Nadine
(86) Numéro de dépôt international: PCT/EP2009/066994
(87) Numéro de publication internationale: WO 2010/066899

(56) Documents cités:
- WO-A-01/43797
- US-A- 4 080 958
- US-A- 4 240 409
- US-A- 6 030 335

## Description

La présente invention concerne un dispositif médical pulsatile destiné à être utilisé avec la chirurgie extra-corporelle ainsi qu'avec d'autres systèmes d'assistance cardiaque ayant comme source une pompe pneumatique, ou électronique. Ce dispositif permet, par exemple, d'obtenir une perfusion pulsatile régulière dans l'organisme d'un patient soumis à une chirurgie cardiaque à coeur ouvert, ou atteint d'une défiance cardiaque.

Le système cardiovasculaire est un circuit hydraulique fermé, sous pression, tapissé intérieurement par les cellules endothéliales. L'endothélium est soumis continuellement aux forces tangentielles du stress de cisaillement (shear stress) qui sont indispensables au maintien de la fonction endothéliale comprenant le tonus vasculaire par la synthèse d'oxyde nitrique (NOS), la coagulation du sang, la réponse inflammatoire, l'athérosclérose, l'angiogénèse et l'apoptose.

La circulation extra-corporelle (en abrégé CEC) est un dispositif médical utilisé en remplacement du coeur et des poumons dans les blocs opératoires, les laboratoires de cathétérisme cardiaque ou les unités de soins intensifs, pour des patients en pédiatrie ou adultes.

En temps que source d'énergie, la console de CEC est un modèle mathématique conçu à partir de lois physiques régissant le déplacement d'un fluide dans un circuit fermé. Ce circuit est concrètement composé d'une pompe, d'un échangeur thermique, d'un débit-mètre, d'un analyseur des gaz et d'électrolytes sanguins, d'un enregistreur de pression ainsi que de matériel biocompatible comme les tubulures, les canules artérielle et veineuse, le réservoir veineux, l'oxygénateur, le filtre artériel.

Habituellement une pompe centrifuge ou péristaltique est utilisée comme pompe de tête artérielle et quatre autres pompes péristaltiques sont utilisées pour l'aspiration de la cardiotomie, la circulation des chambres cardiaques, l'administration de la cardioplégie et une pompe de secours.

La CEC est nécessaire pour maintenir la perfusion des organes et les fonctions métaboliques lors de la cardioplégie chirurgicale ou pour assister le muscle cardiaque défaillant jusqu'à sa récupération ou comme relais avant la transplantation.

Malgré les progrès faits dans le domaine de la CEC depuis son apparition au début des années 1950, celle-ci présente encore des inconvénients, responsables de désordres hémodynamiques post-opératoires.

Les surfaces artificielles de la CEC activent la coagulation et les plaquettes avec une grande probabilité de formation de caillots à l'intérieur du circuit à l'origine de situations délicates comme des saignements post-opératoires et des désordres biochimiques et électrolytiques.

Le flux laminaire de la CEC conventionnelle dégage l'endothélium des stimuli des forces tangentielles du stress de cisaillement causant un syndrome de dysfonction endothéliale responsable de l'activation de la cascade de complément, de la réponse inflammatoire, de l'apoptose et des désordres hémodynamiques, spécialement chez les nouveaux-nés et les enfants.

La circulation d'un fluide dans un circuit hydraulique fermé (transfert d'énergie avec pertes de charge par flottement) dépend de la source d'énergie, de la géométrie des tubulures, de la viscosité et la densité du fluide.

Les trois principales lois physiques impliquées dans ce mécanisme sont : la loi de Newton sur la force de cisaillement (1668), la troisième équation de Bernoulli sur les pertes énergétiques (1738) et les nombres de Reynolds (1880) qui définissent la densité et le profil de déplacement du liquide.

La CEC est le produit de théories s'appliquant aux fluides Newtoniens à viscosité constante, se déplaçant dans un circuit fermé avec une géométrie des tubulures fixe.

La circulation cardiovasculaire contient un fluide non Newtonien, se déplaçant dans des vaisseaux de géométrie variable.

Il y a donc confrontation entre deux circuits hydrauliques différents et difficulté à les adapter fonctionnellement et à les faire travailler en harmonie.

Les résistances vasculaires de la circulation cardiovasculaire sont principalement contrôlées par la sécrétion de monoxyde d'azote (NO), dépendante de la stimulation endothéliale par la force de cisaillement. Par contre, dans la CEC, les résistances sont plus liées au type de tubulure, à l'oxygénateur, à la canule aortique, à la viscosité et au flux laminaire ou turbulent.

Ceci signifie, en pratique, quantifier la force motrice circulatoire, qu'elle soit naturelle (le coeur) ou artificielle (la CEC). Cette force motrice circulatoire dépend de sa capacité à déplacer le sang avec des résistances faibles et donc de maintenir le tonus vasculaire et les fonctions endothéliales par la force de cisaillement (Shear Stress), d'un flux pulsatile régulier avec une pression différentielle (pulse pressure), physiologique.

Pour surmonter ces inconvénients de la CEC classique, des stratégies thérapeutiques variées ont été appliquées :
- l'utilisation de médicaments tels que anticoagulants, inotropes, vasodilatateurs, hormones, électrolytes, sang, plaquettes ou autres substituts qui n'ont pas complètement résolu les problèmes hémodynamiques et qui présentent leurs propres effets indésirables;
- la CEC en normothermie : elle est de plus en plus répandue, en remplacement de la CEC en hypothermie. La normothermie rend le sang pratiquement newtonien ce qui permet de limiter les pertes de charge et la réponse inflammatoire, particulièrement à l'intérieur du circuit de CEC. Cependant, son action sur le coeur et les vaisseaux est toujours soumise à question car le myocarde est protégé par les injections cardioplégiques et la microcirculation est facilitée par l'hémodilution (effet Fahraeus-Lindquist) ;
- la chirurgie sur coeur battant, sans CEC. A côté de ses effets bénéfiques sur l'hémodynamique post-opératoire, c'est une technique délicate, réservée aux patients peu complexes. Elle nécessite du matériel spécifique pour immobiliser la partie opérée, ce qui en fait une méthode coûteuse ;
- la CEC pulsatile : pour conserver la stimulation du stress de cisaillement et conserver la fonction endothéliale pendant l'intervention, un mode pulsatile de perfusion a été utilisé par différents groupes pendant les trois dernières décennies. Actuellement, les nouvelles générations de CEC pulsatile utilisent des pompes péristaltiques ou centrifuges modifiées pour générer un flux de perfusion pulsatile. Ceci nécessite habituellement une double tête de pompe artérielle, une en amont et une en aval de l'oxygénateur, pour limiter son effet obstructif. Ceci nécessite donc un matériel spécifique, de haute technologie, à coût élevé, avec des courbes de pression induite médiocres. Elles sont donc peu diffusées.

On peut noter que pratiquement toutes ces machines pulsatiles ont été évaluées selon les équations de pertes énergétiques de Bernoulli et la perfusion de la microcirculation lors de la CEC dépend de l'hémodilution plus que du type de force motrice utilisée. De même, il semble paradoxal de pulser un fluide comme le sang avec ses composants très fragiles, dans un long tuyau rigide, de faible diamètre, quelque soit son matériau, chlorure de polyvinyle (PVC) ou silicone, avec ses effets indésirables de micro emboles.

Une autre méthode pour obtenir une certaine pulsation lors de la CEC conventionnelle (avec flux continu) est l'adjonction d'un ballon de contre-pulsion intraaortique, introduit invasivement jusqu'à l'aorte thoracique. Bien que peu coûteuse et corrélée à la CEC pulsatile, cette méthode demeure invasive avec des complications vasculaires spécialement chez l'enfant et l'adulte athérosclérotique.

En conclusion, ces méthodes sont habituellement évaluées par l'amélioration de la perfusion d'organes localisés comme le territoire splanchnique, rénal, mais fournissent une protection faible des organes distaux comme la circulation cérébrale. Cela crée des zones de cisaillement turbulent ou de stress de Reynolds, avec des vortex aux abords de la paroi des vaisseaux, vortex créés par la rencontre de deux flux opposés, le flux laminaire de la CEC et le flux pulsatile du ballon, ce qui compromet l'endothélium régional.

Les méthodes citées ci-dessus ne résolvent donc pas les effets indésirables de la CEC.

Les inventeurs ont proposé une solution dans la demande internationale WO/2008/000110. Cette solution consiste en un tube pulsatile comprenant une partie dite à double lumière dans laquelle la tubulure gonflable a été intégré de façon mira-lumen ou extra-lumen.

La présente invention consiste en une amélioration de ce tube pulsatile à double lumière.

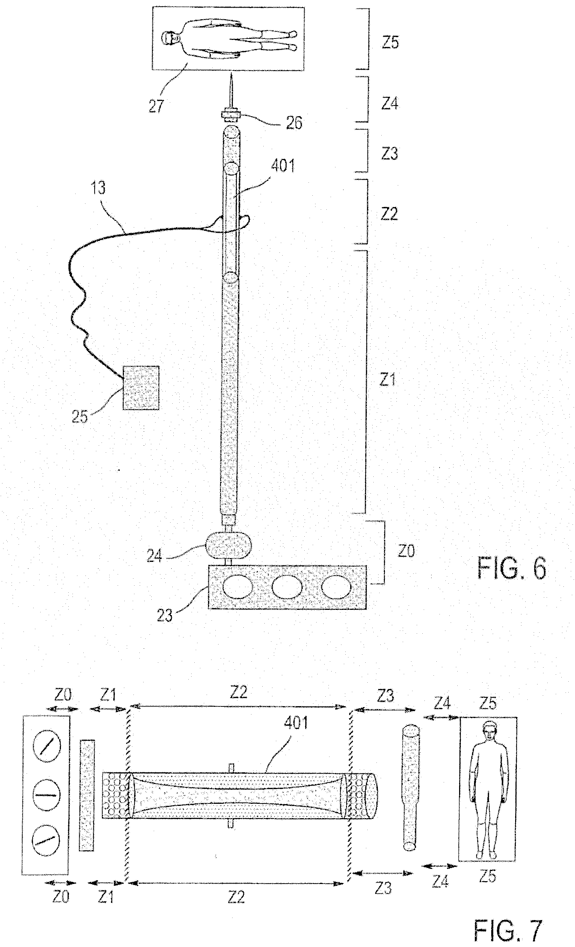

Le but du dispositif (ou tube) médical pulsatile amélioré de la présente invention est de fournir un dispositif générant un flux plus homogène, de minimiser les pertes de charge du flux pulsatile, spécialement dans la zone pulsatile effective (voir la figure 6), de diffuser l'utilisation des systèmes d'assistance cardiaque pulsatiles en tant que méthode physiologique, à bas coût, facilement disponibles et adaptables aux sources d'énergie disponibles dans les services médicaux et chirurgicaux pour les patients de pédiatrie aussi bien que pour les adultes.

Le dispositif médical pulsatile de l'invention est adaptable à tous les types de systèmes d'assistance cardiaque en flux continu et peut être à usage unique.

L'invention est définie par le jeu de revendications ci-joint. Le dispositif médical pulsatile est un dispositif permettant la circulation d'un flux sanguin et comprenant :
- un tuyau externe présentant une paroi interne, une paroi externe et deux extrémités, une extrémité destinée à être connectée à une machine de type CEC et une extrémité destinée à être connectée au corps du patient ;
- un tuyau interne, inséré dans ledit tuyau externe, présentant une paroi interne, une paroi externe et deux extrémités fixées, sur toute leur périphérie, au tuyau externe, sur toute sa périphérie, le flux sanguin circulant au travers dudit tuyau interne ;
- la paroi externe du tuyau interne et la paroi interne du tuyau externe définissant un espace destiné à être rempli de fluide, ledit espace étant relié par un port connecteur à un appareil permettant de créer un (des) gonflements/dégonflements dudit espace entraînant la création de pulsation(s) du flux sanguin.

La circulation d'un seul flux sanguin sur lequel est appliqué de manière directe une pulsation permet de créer un flux sanguin beaucoup plus homogène et régulier que celui de l'art antérieur. En effet, dans l'art antérieur la rencontre d'un flux laminaire venant de la CEC avec un flux pulsatile venant, par exemple, d'un ballon de contre-pulsion intra-aortique, crée des vortex qui ne se créent plus dans le dispositif de l'invention. De même les pertes de charge sont diminuées.

Dans un premier mode de réalisation de l'invention, les extrémités du tuyau interne du dispositif médical pulsatile de l'invention sont fixées sur la paroi interne du tuyau externe.

Les extrémités du tuyau interne sont fixées et scellées par une technique équivalente à celle des stents endovasculaires, des ombrelles interventionnelles. On peut ainsi l'utiliser sur de plus grandes longueurs de tuyaux avec les avantages de diminuer les pertes énergiques en supprimant les multiplications de raccords.

Dans un deuxième mode de réalisation de l'invention, le tuyau interne est de longueur sensiblement identique à celle du tuyau externe et les extrémités du tuyau interne du dispositif médical pulsatile de l'invention sont fixées sur la paroi externe du tuyau externe.

Les extrémités du tuyau interne sont retournées et scellées extérieurement sur la paroi externe du tuyau externe. On évite ainsi de manière avantageuse le risque d'embolie gazeuse en cas de fuite de fluide, grâce à un système extracorporel.

Dans une forme d'exécution particulière de l'invention, une valve est fixée à une ou aux deux extrémités dudit tuyau interne du dispositif médical pulsatile de l'invention.

La présente de cette (ces) valve(s) empêche tout reflux du flux sanguin qui ne peut alors circuler que dans un sens. Ce flux sanguin monodirectionnel réduit les effets de vortex et les pertes de charge.

Dans un mode de réalisation particulier, ledit espace défini entre la paroi externe du tuyau interne et la paroi interne du tuyau externe du dispositif médical pulsatile de l'invention est pré-rempli de fluide.

Du fait de ce pré-remplissage, l'utilisation du dispositif s'avère moins coûteuse en énergie car l'appareil permettant de créer une pulsation ne doit plus fournir que le fluide nécessaire à comprimer cet espace déjà rempli (et n'a pas à le remplir en plus) pour exercer une pression sur le flux sanguin. Dans ce mode de réalisation, le fluide exerce une pression sur la paroi externe du tuyau externe qui fléchit et dans ce cas, le tuyau externe ne peut être fait d'un matériau rigide.

Dans une forme d'exécution particulière, l'appareil permettant de créer une pulsation, du dispositif médical pulsatile selon l'invention, comprend :
- une poche, adaptée à être remplie de fluide, et
- un moyen de compression de poche, adapté à comprimer ladite poche de manière pulsée,
le port connecteur reliant ladite poche audit espace et permettant la circulation du fluide entre ledit espace et ladite poche. Un tel appareil est notamment décrit dans la demande FR 08 01818 appartenant également au demandeur.

Dans une autre forme de réalisation, l'appareil d'application d'une pression pulsatile déterminée sur un dispositif médical selon l'invention comprend :
- un moyen de prélèvement adapté à prélever du fluide depuis une source de fluide en flux continu sous haute pression ;
- un moyen de transformation adapté à transformer ledit fluide en un fluide en flux pulsatile sous basse pression ;
- au moins un moyen d'application pour appliquer ledit fluide, en flux pulsatile sous basse pression sur ledit dispositif médical, le port connecteur reliant ledit moyen d'application audit espace ; et
- un moyen d'évacuation dudit fluide.

Un tel appareil est notamment décrit dans la demande FR 08 02871 appartenant également au demandeur.

De préférence, le dispositif médical pulsatile comprend également la poche adaptée à être remplie de fluide. Celle-ci peut être raccordée audit dispositif pulsatile lors de sa fabrication et/ou réalisée d'une seule pièce avec celui-ci, de sorte qu'il est possible de mettre à disposition, un dispositif médical pulsatile pourvu d'une poche, un même moyen de compression de poche pouvant être utilisé pour plusieurs dispositifs selon l'invention munis respectivement de leur poche.

Ainsi, le dispositif médical pulsatile selon l'invention que l'on peut appeler également tubulure, est valvé, pré-rempli et associé avec des extrémités réalisées en matériels biocompatibles et il peut ainsi être également fixé par des sutures directes aux tissus du coeur et aux vaisseaux. Ce dispositif qui est alors associé avec un appareil permettant de créer une pulsation tel que décrit ci-dessus constituera un système d'assistance ventriculaire complet à part entière.

Ce système est de faible coût et d'utilisation simple. Il ne nécessite pas de source de pression coûteuse comme une pompe pour ballon intra-aortique.

L'invention concerne également un ensemble médical pulsatile comprenant :
- une zone dite Z0 comprenant une pompe de type CEC et un oxygénateur ;
- une zone dite Z1 comprenant une première extrémité du dispositif médical pulsatile selon l'invention ;
- une zone dite Z2 comprenant un dispositif médical pulsatile selon l'invention;
- une zone dite Z3 comprenant la deuxième extrémité du dispositif médical pulsatile selon l'invention ; et
- une zone dite Z4 comprenant une canule aortique.

Cet ensemble médical pulsatile est un système d'assistance cardiaque pulsatile qui représente une méthode physiologique, à bas coût, facilement disponible et adaptable aux sources d'énergie disponibles dans les services médicaux et chirurgicaux pour les patients de pédiatrie aussi bien que pour les adultes.

Pour analyser les paramètres définissant le prototype selon les lois physiques, défendant la supériorité du phénomène produit, avec des pertes d'énergie minimisées, on a donc divisé le circuit de la circulation extracorporelle (CEC) en 6 zones :
**Z0** : comprenant une pompe de type CEC et un oxygénateur dans laquelle existe le phénomène initial provoqué par la CEC conventionnelle.
**Z1** : comprenant une première extrémité du dispositif médical pulsatile selon l'invention et correspond à la zone avec un flux continu régulier depuis l'oxygénateur.
**Z2** : comprenant un dispositif médical pulsatile selon l'invention, elle représente la partie pulsatile du prototype, propulsant les couches stagnantes de la périphérie vers le centre, en diminuant les effets traumatiques sur les composants du sang (globules rouges).
**Z3:** comprenant la deuxième extrémité du dispositif médical pulsatile selon l'invention, elle représente la zone effective du flux pulsatile, c'est la zone la plus courte du circuit afin de conserver au maximum l'énergie pulsatile.
**Z4**: Elle représente la canule aortique.
**Z5**: Tissus irrigués du patient

Le flux sanguin doit être transféré de la machine de circulation extracorporelle (CEC) jusqu'aux organes perfusés du patient (de Z0 à Z5) en minimisant les pertes de charges énergétiques.

En résumé l'ensemble selon l'invention engendre moins de pertes énergétiques par divergence du flux et moins d'effets hémolytiques, car ils ne se produisent que de la Z3 à Z5, alors que pour les systèmes de CEC actuels, pulsatiles ou non, les pertes s'étalent Z0 à Z5 avec le plus de résistances.

Dans une forme d'exécution particulière, le dispositif médical pulsatile selon l'invention dudit ensemble médical pulsatile est placé entre l'oxygénateur et la canule aortique.

Dans une autre forme d'exécution particulière, la distance représentée par la zone dite Z3 dudit ensemble médical pulsatile selon l'invention est réduite au minimum.

De préférence, la partie comprenant les tuyaux interne et externe sera adaptée aux diverses dimensions des systèmes d'assistance cardiaque en termes de longueur, de diamètre, de volume et de fréquence de gonflage.

L'invention sera mieux comprise par référence aux dessins annexés dans lesquels :
- la figure 1 est une représentation schématique d'un premier mode de réalisation, en coupe longitudinale, d'un dispositif médical pulsatile selon l'invention ;
- la figure 2 est une représentation schématique d'un deuxième mode de réalisation, en coupe longitudinale, d'un dispositif médical pulsatile selon l'invention ;
- la figure 3 est une représentation schématique d'un troisième mode de réalisation, en coupe longitudinale, d'un dispositif médical pulsatile selon l'invention ;
- la figure 4 est une représentation schématique d'un exemple d'appareil permettant de créer une pulsation au travers du dispositif médical pulsatile selon l'invention ;
- les figures 5a et 5b sont une représentation schématique d'un procédé de fixation du tuyau interne au tuyau externe, dans le mode de réalisation de la figure 1 ;
- la figure 6 est une représentation schématique d'un ensemble médical pulsatile selon l'invention adapté à un patient ;
- la figure 7 est une représentation schématique des pertes de charge de l'ensemble médical pulsatile de la figure 6 ;
- les figures 8a, 8b et 8c sont des représentations schématiques de circuits représentant un ensemble médical pulsatile selon l'invention appliqué à une résistance simulant un patient, résistance reliée, par retour, à l'ensemble médical pulsatile selon l'invention, créant un circuit fermé.

Dans les dessins, les mêmes chiffres (unité et dizaine) de référence désignent des structures correspondantes d'une figure à l'autre, le chiffre des centaines distinguant les différentes variantes.

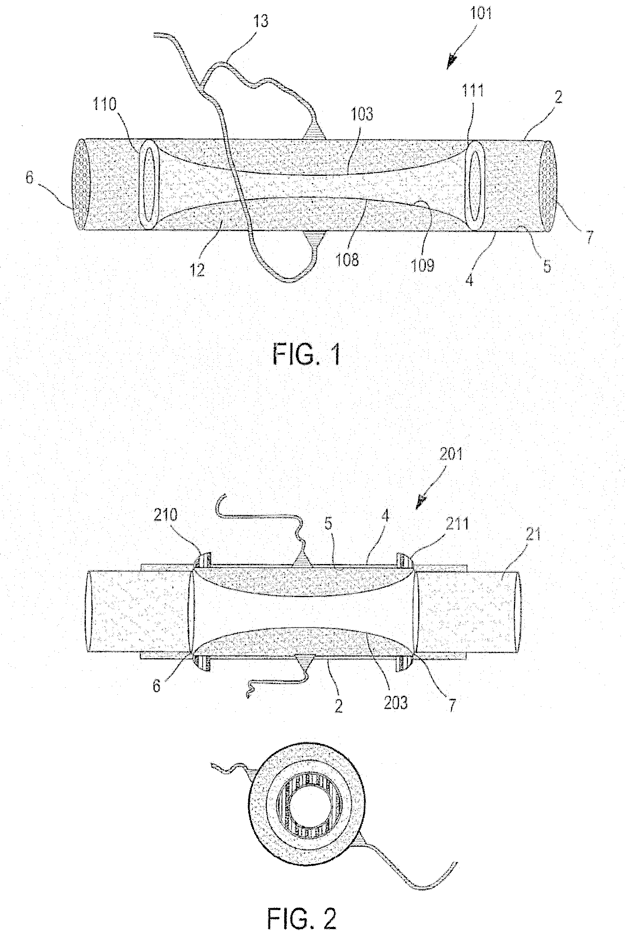

La figure 1 représente un premier mode de réalisation du dispositif médical pulsatile 101 de l'invention. Ce dispositif comprend un tuyau externe 2 dans lequel est inséré un tuyau interne 103. Le tuyau interne 103 est un tuyau compressible fabriqué en matériau biocompatible, flexible, comme, par exemple, le polyuréthane. Le tuyau externe 2 est plus rigide, fabriqué, par exemple en chlorure de polyvinyle (PVC) et sert de coque externe.

Le tuyau externe 2 présente une paroi externe 4, une paroi interne 5, une première extrémité 6 destinée à être connectée, par exemple, à une machine de type CEC et une deuxième extrémité 7 destinée à être connectée au corps d'un patient. Le tuyau interne 103 présente également une paroi externe 108 et une paroi interne 109 ainsi que deux extrémités 110 et 111. Le tuyau interne 103, sur la figure 1, est de longueur inférieure au tuyau externe 2, la zone où les deux tuyaux 2 et 103 sont présents est une zone dite « à double lumière ». Dans le mode de réalisation de la figure 1, les extrémités 110 et 111 du tuyau interne 103 sont fixées sur toute leur périphérie à la paroi interne 5 du tuyau externe 2, sur toute sa périphérie. Ainsi, un espace 12, appelé réservoir de fluide, est créé entre la paroi interne 5 du tuyau externe 2 et la paroi externe 108 du tuyau interne 103 dans la zone de double lumière. Un port connecteur 13 est destiné à être fixé d'une part audit espace 12 et d'autre part à un appareil permettant de créer une pulsation (non représenté sur la figure 1). L'appareil permettant de créer une pulsation peut être, par exemple, une source de pression pneumatique rythmique ou un système rythmique électronique. Le port connecteur 13 peut être fixé à l'espace 12 par un ou des orifices présents sur le tuyau externe 2. Dans une forme d'exécution, les orifices peuvent être munis de clapets fermés lorsque le port connecteur n'est pas fixé et qui s'ouvrent sous la pression du fluide arrivant du port connecteur 13 lorsque celui-ci est fixé. Sur la figure 1, le port connecteur 13 est fixé en deux positions diamétralement opposées du tuyau externe 2 dans la zone à double lumière, mais il est bien évident qu'une fixation en une ou plusieurs positions est envisageable.

En fonctionnement, le dispositif médical pulsatile 101 est relié d'une part à une machine de type CEC (côté extrémité 6) et d'autre part au corps du patient (côté extrémité 7). Puis, le port connecteur 13 est fixé d'une part au dispositif médical pulsatile 101, au niveau de l'espace 12, et d'autre part à un appareil permettant de créer une pulsation, cette fixation du port connecteur 13 pouvant se faire postérieurement à la mise en circulation du flux sanguin. Le flux sanguin est alors mis en circulation : il va de la machine de type CEC vers le corps du patient en passant au travers du tuyau externe 2 puis du tuyau interne 103, dans la zone à double lumière puis de nouveau au travers du tuyau externe 2 jusqu'au corps du patient. Le flux sanguin est alors continu. Pour créer une pulsation, l'appareil permettant de créer une pulsation est mis en fonctionnement : cet appareil fournit régulièrement un fluide (par exemple un gaz tel que de l'hélium ou du dioxyde de carbone) qui circule au travers du port connecteur 13 jusque dans l'espace 12. Arrivé dans l'espace 12, le fluide remplit cet espace jusqu'à comprimer la paroi externe 108 du tuyau interne 103, la paroi interne 5 du tuyau externe 2 restant rigide. Il s'ensuit une compression du tuyau interne 103 créant une pulsation sur le flux sanguin circulant au travers dudit tuyau interne 103. Un système de commande peut être adjoint audit appareil permettant de créer une pulsation, un opérateur pouvant alors programmer la fréquence des pulsations ainsi que leur force.

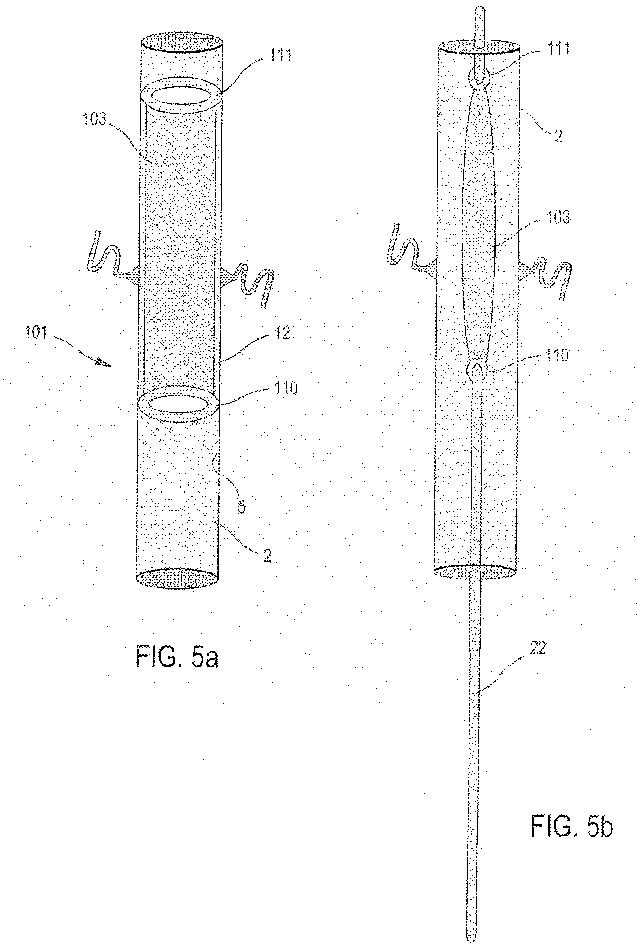

Les figures 5a et 5b représente un exemple de procédé de mise en place du tuyau interne 103 à l'intérieur du tuyau externe 2 selon le mode de réalisation de la figure 1. Le tuyau interne 103 est introduit dans le tuyau externe 2 par un mandrin 22 de manière classique en soi. Une fois le tuyau interne 103 détaché du mandrin 22, le mandrin 22 est retiré et ce retrait entraîne le déploiement des extrémités 110 et 111 du tuyau interne 103, ces extrémités 110 et 111 venant se caler contre la paroi, interne 5 du tuyau externe 2. Le tuyau interne 103 est alors fixé à chacune de ses extrémités 110 et 111 par une ombrelle déployable de manière classique.

Dans une forme d'exécution particulière de l'invention, l'espace 12 peut être pré-rempli avec un fluide inerte. Dans cette tonne d'exécution l'appareil permettant de créer une pulsation doit alors fournir une quantité de fluide inférieure à celle nécessaire dans le mode de réalisation décrit ci-dessus pour entraîner une pulsation. En effet, l'étape de remplissage de l'espace 12 est déjà effectuée. Cette forme d'exécution du dispositif médical pulsatile de l'invention est alors plus compacte. Elle doit naturellement être apte à la stérilisation et à l'emballage.

La figure 2 représente un deuxième mode de réalisation du dispositif médical pulsatile 201 identique à celui de la figure 1 excepté que le tuyau interne 203 est aussi long que le tuyau externe 2 d'où une autre configuration possible des extrémités 210 et 211 du tuyau interne 203. En effet, ces extrémités ne sont pas fixées sur la paroi interne 5 du tuyau externe 2 mais sur la paroi externe 4 du tuyau externe 2, par retournement. Ce dispositif médical pulsatile 201 est alors fixé, à ses deux extrémités 6/210 et 7/211 à des ports connecteurs 21 de CEC standards par des raccords de façon à éviter des accidents intravasculaires en cas de fuite de fluide.

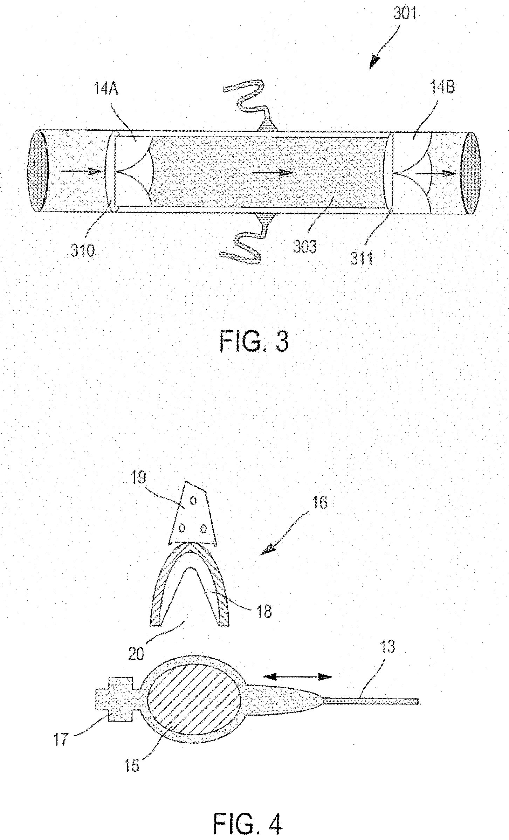

La figure 3 représente un troisième mode de réalisation du dispositif médical pulsatile 301 dans lequel deux valves de type monodirectionnel 14A et 14B ont été placées aux extrémités 310 et 311 du tuyau interne 303.

La présence de ces valves 14A et 14B permet d'obtenir un flux sanguin monodirectionnel (schématisé par des flèches), réduisant les effets de vortex et les pertes de charge.

La figure 4 représente un exemple d'appareil permettant de créer une pulsation au travers du dispositif médical pulsatile selon l'invention. Cet appareil comprend :
- une première partie incluant une poche 15 remplie de fluide, reliée à une extrémité au port connecteur 13 et à son autre extrémité à une valve 17 anti-reflux, permettant de remplir la poche 15 de fluide ou de la vider, et
- une deuxième partie constituée d'un moyen de compression 16 de ladite poche 15 comprenant un compartiment compresseur de poche 18 et une commande 19, par exemple électromécanique, dudit compartiment compresseur 18. Le compartiment compresseur 18 présente un évidemment 20.

En fonctionnement, le dispositif médical pulsatile de l'invention est placé entre le corps d'un patient et une machine de type CEC. Le port connecteur 13 est fixé d'une part au dispositif médical pulsatile de l'invention (au niveau de l'espace 12) et d'autre part à la poche 15. La poche 15 est remplie de fluide par ouverture de la valve 17 (cette opération pouvant avoir été effectuée avant la connexion du port connecteur 13 à la poche 15). La poche 15 est ensuite placée dans l'évidement 20 du compartiment compresseur 18 commandé par une commande électromécanique 19. Selon les instructions reçues par la commande électromécanique 19, une fréquence précise de compression/décompression de la poche 15 va se mettre en place, la fréquence pouvant être de 10 à 300 compressions par minute. La compression de la poche 15 entraîne un flux de fluide en direction de l'espace 12 qui se gonfle (comprimant le tuyau interne et créant une pulsation du flux sanguin) et la décompression de la poche 15 entraîne un appel du fluide de l'espace 12 vers la poche 15, ce qui dégonfle l'espace 12. La double flèche de la figure 4 illustre le trajet du fluide. On obtient alors un mouvement pulsatile du fluide et donc un gonflement/dégonflement pulsatile de l'espace 12 entraînent la formation de pulsation(s) dans le flux sanguin circulant dans le tuyau interne. On peut envisager un moyen de compression 16 comprenant un système permettant de synchroniser la fréquence de compressions (donc de pulsations) sur la fréquence de battements du coeur du patient. Ce moyen de compression 16 est d'un faible coût et, de par son encombrement réduit, portable.

Cet exemple concernant un moyen de compression 16, est particulièrement adapté au mode de réalisation du dispositif médical pulsatile de l'invention dans lequel l'espace 12 est pré-rempli de fluide.

La figure 6 est un schéma de liaison machine de CEC - patient rencontré en chirurgie cardiaque ou lors de l'utilisation d'un système d'assistance cardiaque. Cette liaison est un circuit divisé en 6 zones. La zone Z0 comprend la pompe de type CEC 23 et un oxygénateur 24, la zone Z1 comprend une première extrémité du dispositif médical pulsatile 401 (pouvant être l'un quelconque des différents modes de réalisation cités ci-dessus), la zone Z2 comprend le dispositif médical pulsatile 401 relié, par l'intermédiaire du port connecteur 13, à un appareil 25 permettant de créer une pulsation, la zone Z3 comprend la deuxième extrémité du dispositif médical pulsatile 401 (c'est la zone pulsatile effective) et la zone Z4 comprend une canule aortique 26. En incluant également les organes perfusés 27 du patient dans une zone Z5 on obtient un circuit hydraulique fermé, sous pression.

La figure 7 est un schéma des pertes de charge du circuit tel que défini ci-dessus. Les pertes de charge sont toujours présentes en Z0 et Z4, positions classiques de l'oxygénateur 24 et de la canule aortique 26, mais ces pertes existent également en Z5 avec la vasoconstriction causée par le flux laminaire ou la perfusion pulsatile inadéquate. Les trois zones Z1, Z2 et Z3 sont des zones qui, grâce au dispositif médical pulsatile 401, présentent des pertes de charge minimales. En Z1, la géométrie du dispositif médical pulsatile 401, ses limites et sa position en aval de l'oxygénateur 24 permet un flux laminaire avec de faibles résistances. En Z2, la compression du tuyau interne lors d'une pulsation déplace les couches périphériques du flux sanguin vers le centre sans créer de turbulences et sans traumatiser les composants du sang (globules rouges). Le flux pulsatile effectif commence donc uniquement en Z3 grâce au dispositif de l'invention. La distance entre le dispositif médical pulsatile et les organes perfusés (soit les zones Z3, Z4 et Z5) doit être minimale (en d'autres termes la zone Z3 doit être minimale puisque les deux autres zones ne peuvent être réduites) pour conserver l'impulsion pulsatile avec le minimum de pertes de charge.

En plus de pertes de charge rendues minimes dans les zones Z1, Z2 et Z3 grâce au dispositif médical pulsatile de l'invention, on peut envisager de réduire les pertes de charge :
- à l'extrémité de la canule aortique 26 (zone Z4) en représentant un diffuseur divergent avec perte de charge qui sera graduellement corrigée par la paroi artérielle selon la loi de Hagen Poiseuille ;
- en Z5, par un flux, pulsatile adapté grâce au dispositif de l'invention permettant une vasodilatation des organes perfusés par augmentation de la sécrétion de (NOS).

Le décompte des pertes de charge est donc grâce au dispositif de l'invention fait en Z3, Z4 et Z5, alors que dans la CEC classique, pulsatile ou non, elles se produisent de Z0 à Z5 couvrant donc trois zones supplémentaires. Le dispositif ne nécessite pas une double pompe de perfusion, ni un oxygénateur spécial à basse résistance, contrairement aux autres CEC pour diminuer leur perte de charge. Pour minimiser les pertes de charge, il est préférable de fabriquer le dispositif médical pulsatile de l'invention en une seule pièce. Le dispositif entraîne moins de turbulences aux extrémités, moins de diffuseurs divergents, cause de pertes de charge et moins d'effets hémolytiques.

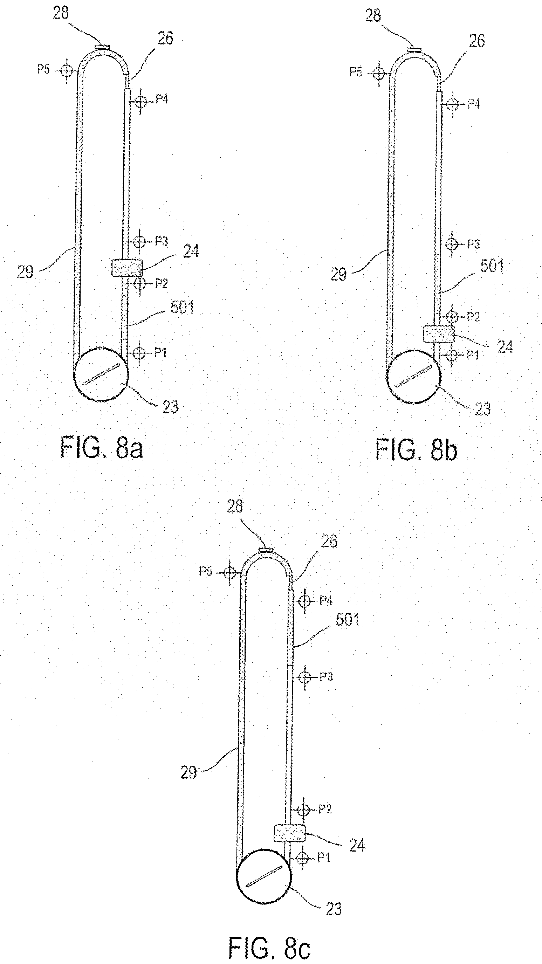

Les figures 8a, 8b et 8c sont des représentations schématiques de modélisations du circuit machine de CEC - patient de la figure 6. Dans ces modélisations, les organes perfusés du patient sont simulés par une résistance 28. Dans ces trois variantes de circuit, on retrouve les mêmes éléments que ceux des figures 6 et 7, à savoir, la pompe de type CEC 23 [le circuit CEC étant en PVC et de 0,63 cm de diamètre (1/4 de pouce)] et l'oxygénateur 24 (avec filtre), le dispositif médical pulsatile selon l'invention 501 (pouvant être l'un quelconque des différents modes de réalisation cités ci-dessus), la canule aortique 26 (de taille 14) et une résistance 28 simulant la résistance vasculaire en amont des organes perfusés. Pour obtenir un circuit fermé et, ainsi, mimer correctement le flux sanguin, un retour en silicone 29 est prévu, reliant la résistance 28 à la pompe de type CEC 23. Du fluide a été mis en circulation dans ce circuit (dans le sens pompe CEC/oxygénateur/résistance) et la pulsation du dispositif médical selon l'invention 501 a été mise en marche. Les pressions ont alors été mesurées en P1, P2, P3, P4 et P5 (emplacements fixes dans le circuit).

La différence entre les trois variantes de modélisation des figures 8a, 8b et 8c réside dans la position du dispositif médical pulsatile 501 : en amont de l'oxygénateur à la figure 8a, en aval de l'oxygénateur aux figures 8b et 8c (la distance entre le dispositif 501 et la résistance 28, simulant les organes perfusés du patient, étant minimale à la figure 8c).

Les résultats de ces mesures de pression (en mmHg) se trouvent dans les tableaux ci-dessous.

### Figure 8c

| Pression | Fixe* | Pulsatile** |
|---|---|---|
| P1 | 31 | 160 |
| P2 | 28 | 150 |
| P3 | 27 | 156 |
| P4 | 24 | 132 |
| P5 | 19 | 93 |

| | | |
|---|---|---|
| * dispositif 501 non soumis à des pulsations ** dispositif 501 soumis à des pulsations | | |

### Figure 8b

| Pression | Fixe | Pulsatile |
|---|---|---|
| P1 | 32 | 128 |
| P2 | 27 | 119 |
| P3 | 26 | 183 |
| P4 | 17 | 129 |
| P5 | 18 | 72 |

| | | |
|---|---|---|
| * dispositif 501 non soumis à des pulsations ** dispositif 501 soumis à des pulsations | | |

### Figure 8a

| Pression | Fixe | Pulsatile |
|---|---|---|
| P1 | 47 | 234 |
| P2 | 28 | 121 |
| P3 | 21 | 92 |
| P4 | 17 | 53 |
| P5 | 19 | 51 |

| | | |
|---|---|---|
| * dispositif 501 non soumis à des pulsations ** dispositif 501 soumis à des pulsations | | |

Les pertes de charge enregistrées sont les plus réduites dans la configuration de la figure 8c c'est-à-dire là où le dispositif pulsatile médical de l'invention 501 est placé en aval de l'oxygénateur 24 mais à une distance minimale des organes perfusés du patient.

Il est bien entendu que l'invention n'est pas limitée aux formes d'exécution décrites et représentées. Ainsi, par exemple, un dispositif médical pulsatile selon l'invention peut présenter des valves et être relié à un appareil permettant de créer une pulsation du type de celui décrit dans la figure 4.

## Revendications

1. Dispositif médical pulsatile (101 ; 201 ; 301 ; 401 ; 501) placé entre le corps d'un patient et une machine de type CEC, et permettant la circulation d'un flux sanguin **caractérisé en ce qu'**il comprend :
- *une canule aortique (26)* ;
- un tuyau externe (2) présentant une paroi interne (5), une paroi externe (4) et deux extrémités (6, 7), une extrémité (6) destinée à être connectée à une machine de type CEC et une extrémité (7) destinée à être connectée au corps du patient *via ladite canule aortique (26)* ;
- un tuyau interne (103 ; 203 ; 303), inséré dans ledit tuyau externe (2), présentant une paroi interne (109), une paroi externe (108) et deux extrémités (110, 111 ; 210, 211 ; 310, 311) fixées, sur toute leur périphérie, au tuyau externe (2), sur toute sa périphérie, le flux sanguin circulant au travers dudit tuyau interne (103 ; 203 ; 303) ; le tuyau interne étant compressible, flexible ;
- la paroi externe (108) du tuyau interne (103 ; 203 ; 303) et la paroi interne (5) du tuyau externe (2) définissant un espace (12) destiné à être rempli de fluide, ledit espace (12) pouvant être relié par un port connecteur (13) à un appareil permettant de créer un (des) gonflements/dégonflements dudit espace (12) entraînant la création de pulsation(s) du flux sanguin ; **en ce que**
ledit port connecteur est connecté audit espace grâce à au moins deux orifices réalisés sur ledit tuyau externe en au moins deux positions diamétralement opposées, et **en ce qu'**une valve (14A, 14B) est fixée à une ou aux deux extrémités (310, 311) dudit tuyau interne (303).

2. Dispositif médical pulsatile (101) selon la revendication 1, **caractérisé en ce que** les extrémités (110, 111) du tuyau interne (103) sont fixées sur la paroi interne (5) du tuyau externe (2).

3. Dispositif médical pulsatile (201) selon la revendication 1, **caractérisé en ce que** le tuyau interne (203) est de longueur sensiblement identique à celle du tuyau externe (2) et les extrémités du tuyau interne (203) sont fixées sur la paroi externe (4) du tuyau externe (2).

4. Dispositif médical pulsatile (101 ; 201 ; 301 ; 401 ; 501) selon l'une quelconque des revendications précédentes, **caractérisé en ce que** ledit espace (12) est pré-rempli de fluide.

5. Dispositif médical pulsatile (101 ; 201 ; 301 ; 401 ; 501) selon l'une quelconque des revendications précédentes, **caractérisé en ce que** l'appareil permettant de créer une pulsation comprend :
- une poche (15), adaptée à être remplie de fluide, et
- un moyen de compression (16) de poche, adapté à comprimer ladite poche (15) de manière pulsée,
- le port connecteur (13) reliant ladite poche (15) audit espace (12) et permettant la circulation du fluide entre ledit espace (12) et ladite poche (15).

6. Dispositif médical pulsatile (101 ; 201 ; 301 ; 401 ; 501) selon l'une quelconque des revendications précédentes, **caractérisé en ce que** l'appareil permettant de créer une pulsation comprend :
- un moyen de prélèvement adapté à prélever du fluide depuis une source de fluide en flux continu sous haute pression ;
- un moyen de transformation adapté à transformer ledit fluide en un fluide en flux pulsatile sous basse pression ;
- au moins un moyen d'application pour appliquer ledit fluide, en flux pulsatile sous basse pression sur ledit dispositif médical, le port connecteur (13) reliant ledit moyen d'application audit espace (12); et
- un moyen d'évacuation dudit fluide.

7. Ensemble médical pulsatile comprenant :
- une zone dite Z0 comprenant une pompe de type CEC (23) et un oxygénateur (24) ;
- une zone dite Z1 comprenant une première extrémité du dispositif médical pulsatile (401) selon l'une quelconque des revendications précédentes ;
- une zone dite Z2 comprenant un dispositif médical pulsatile (401) selon l'une quelconque des revendications précédentes ;
- une zone dite Z3 comprenant la deuxième extrémité du dispositif médical pulsatile (401) selon l'une quelconque des revendications précédentes ; et
- une zone dite Z4 comprenant *la* canule aortique (26) *dudit dispositif médical pulsatile (401).*

8. Ensemble médical pulsatile selon la revendication 7, **caractérisé en ce que** le dispositif médical pulsatile (501) est placé entre l'oxygénateur (24) et la canule aortique (26) *dudit dispositif médical pulsatile.*

## Patentansprüche

1. Pulsierende medizinische Vorrichtung (101; 201; 301; 401; 501), die zwischen den Körper eines Patienten und eine Maschine vom Typ CEC, die die Zirkulation eines Blutstroms ermöglicht, platziert ist, **dadurch gekennzeichnet, dass** sie umfasst:
- eine *Aortakanüle (26),*
- eine externe Röhre (2), die eine interne Wand (5), eine externe Wand (4) und zwei Enden (6, 7), ein Ende (6), das für den Anschluss an eine Maschine vom Typ CEC bestimmt ist, und ein Ende (7), das zum Anschluss an den Körper des Patienten über *die genannte Aortakanüle* (26) bestimmt ist, aufweist;
- eine interne Röhre (103; 203; 303), die in die genannte externe Röhre (2) eingeführt ist und eine interne Wand (109), eine externe Wand (108) und zwei Enden (110; 111; 210; 211; 310; 311) aufweist, die auf ihrer gesamten Peripherie an der externen Röhre (2), auf deren gesamter Peripherie befestigt ist, wobei der Blutstrom durch die genannte interne Röhre (103; 203; 303) zirkuliert; wobei die interne Röhre komprimierbar, flexibel ist;
- wobei die externe Wand (108) der internen Röhre (103; 203; 303) und die interne Wand (5) der externen Röhre (2) einen Raum (12) definieren, der dazu bestimmt ist, mit Flüssigkeit gefüllt zu werden, wobei der genannte Raum (12) durch einen Anschlussport (13) an ein Gerät angeschlossen werden kann, das es erlaubt, ein (oder mehrere) Aufblas- / Luftablassvorgänge des genannten Raums (12) zu generieren, die die Generierung von Pulsierungen des Blutstroms nach sich ziehen; dass der genannte Anschlussport dank wenigstens zwei Öffnungen, die an der genannten externen Röhre in wenigstens zwei diametral entgegengesetzten Positionen realisiert sind, an den genannten Raum angeschlossen ist, und dass ein Ventil (14A, 14B) an einem oder an zwei Enden (310, 311) der genannten internen Röhre (303) befestigt ist.

2. Pulsierende medizinische Vorrichtung (101) gemäß Anspruch 1, **dadurch gekennzeichnet, dass** die Enden (110, 111) der internen Röhre (103) an der internen Wand (5) der externen Röhre (2) befestigt sind.

3. Pulsierende medizinische Vorrichtung (201) gemäß Anspruch 1, **dadurch gekennzeichnet, dass** die interne Röhre (203) eine deutlich identische Länge zu der der externen Röhre (2) hat und die Enden der internen Röhre (203) an der externen Wand (4) der externen Röhre (2) befestigt sind.

4. Pulsierende medizinische Vorrichtung (101; 201; 301; 401; 501) gemäß irgendeinem der voranstehenden Ansprüche, **dadurch gekennzeichnet, dass** der genannte Raum (12) mit Flüssigkeit vorbefüllt ist.

5. Pulsierende medizinische Vorrichtung (101; 201; 301; 401; 501) gemäß irgendeinem der voranstehenden Ansprüche, **dadurch gekennzeichnet, dass** das Gerät, das das Generieren eines Pulsierens ermöglicht, umfasst:
- einen Beutel (15), der geeignet ist, mit Flüssigkeit gefüllt zu werden, und
- ein Kompressionsmittel (16) des Beutels, das geeignet ist, den genannten Beutel (15) pulsierend zu komprimieren,
- wobei der Anschlussport (13) den genannten Beutel (15) mit dem genannten Raum (12) verbindet und die Zirkulation des Stroms zwischen dem genannten Raum (12) und dem genannten Beutel (15) zulässt.

6. Pulsierende medizinische Vorrichtung (101; 201; 301; 401; 501) gemäß irgendeinem der voranstehenden Ansprüche, **dadurch gekennzeichnet, dass** das Gerät, das Generieren eines Pulsierens ermöglicht, umfasst:
- ein Entnahmemittel, das geeignet ist, Flüssigkeit aus einer Flüssigkeitsquelle mit kontinuierlichem Strom unter Hochdruck zu entnehmen;
- Transformationsmittel, die geeignet sind, die genannte Flüssigkeit in eine Flüssigkeit mit einem pulsierenden Strom unter niedrigem Druck zu transformieren;
- wenigstens ein Anwendungsmittel für die Anwendung der genannten Flüssigkeit im pulsierenden Strom unter niedrigem Druck auf die genannte medizinische Vorrichtung, wobei der Anschlussport (13) das genannte Anwendungsmittel mit dem genannten Raum (12) verbindet; und
- ein Austragsmittel der genannten Flüssigkeit.

7. Pulsierende medizinische Gruppe, umfassend:
- einen als Z0 bezeichneten Bereich, umfassend eine Pumpe vom Typ CEC (23) und einen Oxygenator (24);
- einen als Z1 bezeichneten Bereich, umfassend ein erstes Ende der genannten pulsierenden medizinischen Vorrichtung (401) gemäß irgendeinem der voranstehenden Ansprüche;
- einen als Z2 bezeichneten Bereich, umfassend eine pulsierende medizinische Vorrichtung (401) gemäß irgendeinem der voranstehenden Ansprüche;
- einen als Z3 bezeichneten Bereich, umfassend das zweite Ende der pulsierenden medizinischen Vorrichtung (401) gemäß irgendeinem der voranstehenden Ansprüche; und
- einen als Z4 bezeichneten Bereich, umfassend *die* Aortakanüle (26) *der genannten pulsierenden medizinischen Vorrichtung (401)*.

8. Pulsierende medizinische Gruppe gemäß Anspruch 7, **dadurch gekennzeichnet, dass** die pulsierende medizinische Vorrichtung (501) zwischen dem Oxygenator (24) und der Aortakanüle (26) der *genannten pulsierenden medizinischen Vorrichtung* platziert ist.

## Claims

1. Pulsatile medical device (101; 201; 301; 401; 501) placed between the body of a patient and a heart-lung machine of CEC type, enabling the circulation of a blood flow **characterised in that** it comprises:
- *an aortic cannula* (26);
- an external tube (2) presenting an inner wall (5), an outer wall (4) and two ends (6, 7), one end (6) designed to be connected to a heart-lung machine and one end (7) designed to be connected to the body of the patient *by way of the said aortic cannula* (26);
- an inner tube (103; 203; 303) inserted in said outer tube (2), presenting an inner wall (109), an outer wall (108) and two ends (110, 111; 210, 211; 310, 311) attached, along their entire periphery, to the outer tube (2), along its entire periphery, the blood flow circulating through said inner tube (103; 203; 303); the inner tube being compressible, flexible;
- the outer wall (108) of the inner tube (103; 203; 303) and the inner wall (5) of the outer tube (2) defining a space (12) designed to be filled with fluid, said space (12) able to be connected by a connecting port(13) to a device enabling the creation of inflation(s)/deflation(s) of said space (12) provoking the creation of pulse (s) of blood flow; **in that** said connecting port is connected to said space by means of at least two openings on said outer tube and at least two diametrically opposite positions, and **in that** a valve (14A, 14B) is attached to one or two ends (310, 311) of said inner tube (303).

2. Pulsatile medical device (101) according to claim 1, **characterised in that** the ends (110, 111) of the inner tube (103) are attached to the inner wall (5) of the outer tube (2).

3. Pulsatile medical device (201) according to claim 1, **characterised in that** the length of the inner tube (203) is roughly identical to that of the outer tube (2) and the ends of the inner tube (203) are attached to the outer wall (4) of the outer tube (2).

4. Pulsatile medical device (101; 201; 301; 401; 501) according to any of the previous claims, **characterised in that** said space (12) is pre-filled with fluid.

5. Pulsatile medical device (101; 201; 301; 401; 501) according to any of the previous claims, **characterised in that** the device used to create a pulse comprises:
- a pouch (15), adapted to be filled with fluid; and
- a means of compression (16) of the pouch, adapted to compress said pouch (15) in a pulsed manner;
- the connecting port(13) connecting said pouch (15) to said space (12) and enabling the circulation of the fluid between said space (12) and said pouch (15).

6. Pulsatile medical device (101; 201; 301; 401; 501) according to any of the previous claims, **characterised in that** the device used to create a pulse comprises:
- a means of taking adapted to taking the fluid from a source of continuous flow fluid under high pressure;
- a means of transformation adapted to transform said fluid into a pulsatile flow of fluid under low pressure;
- at least one means of application to apply said fluid, into pulsatile flow under low pressure on said medical device, the connecting port(13) connecting said means of application to said space (12); and
- a means of evacuation of said fluid.

7. Pulsatile medical device comprising:
- a zone said Z0 comprising a pump such as a heart-lung machine (23) and an oxygenator (24);
- a zone said Z1 comprising a first end of the pulsatile medical device (401) according to any of the previous claims;
- a zone said Z2 comprising a pulsatile medical device (401) according to any of the previous claims;
- a zone said Z3 comprising a second end of the pulsatile medical device (401) according to any of the previous claims; and
- a zone said Z4 comprising *the aortic cannula* (26) *of said pulsatile medical device* (*401*).

8. Pulsatile medical device according to claim 7, **characterised in that** the pulsatile medical device (501) is placed between the oxygenator (24) and the aortic cannula (26) *of said pulsatile medical device.*
